# EUROPÄISCHE PATENTSCHRIFT

(11) **EP 1 852 074 B1**
(45) Veröffentlichungstag und Bekanntmachung des Hinweises auf die Patenterteilung: **21.01.2015**
(21) Anmeldenummer: 07008547.7
(22) Anmeldetag: 26.04.2007
(51) Int. Cl.: A61B 17/34, A61M 39/06

(54) **Dichtung für eine Trokarhülse sowie derartige Trokarhülse**
Seal for a trocar sheath and such a trocar sheath
Joint pour une gaine de trocart tout comme gaine de trocart de ce type

(30) Priorität: 03.05.2006 DE 102006021974
(43) Veröffentlichungstag der Anmeldung: 07.11.2007
(60) Teilanmeldung: 10180808.7
(73) Patentinhaber: Karl Storz GmbH & Co. KG, 78532 Tuttlingen (DE)
(72) Erfinder: Lang, Dieter, 96342 Stockheim (DE)
(74) Vertreter: Heuckeroth, Volker

(56) Entgegenhaltungen:
- EP-A2- 0 316 096
- EP-A2- 0 567 141
- WO-A-99/42152
- WO-A-03/000145
- DE-A1- 3 737 121
- FR-A1- 2 863 504
- US-A- 5 685 854
- US-A- 5 788 676
- US-A1- 2005 261 661
- US-B1- 6 276 661

## Beschreibung

Die Erfindung betrifft eine Dichtung für eine Trokarhülse, mit einem Dichtungskörper, der in Richtung einer Längsmittelachse des Dichtungskörpers ein erstes axiales Ende und ein zweites axiales Ende und zwischen dem ersten und zweiten Ende ein elastisches Dichtelement aufweist, das in einem bezüglich der Längsmittelachse radial inneren Bereich einen Durchgang für ein Instrument definiert, wobei das Dichtelement einen im Wesentlichen schlauchförmigen Hohlraum aufweist, dessen radial innerer Wandbereich den Durchgang ringförmig umgibt, und wobei der Hohlraum ein Fluid enthält, wobei das Dichtelement einen radial äußeren Wandbereich radial außerhalb des Hohlraums aufweist, dessen Wandstärke größer ist als die Wandstärke des radial inneren Wandbereichs.

Die Erfindung betrifft ferner eine Trokarhülse mit einer derartigen Dichtung.

Eine Dichtung der eingangs genannten Art ist aus dem Dokument DE 37 37 121 A1 bekannt.

Trokare werden auf dem Gebiet der Medizin im Rahmen der minimal-invasiven Chirurgie zum Einführen von Instrumenten in den menschlichen oder tierischen Körper verwendet. Bei einem minimal-invasiven chirurgischen Eingriff wird ein Trokar, der allgemein aus einer Trokarhülse und einem in der Trokarhülse aufgenommenen Trokardorn besteht, zunächst dazu verwendet, einen minimal-invasiven Zugang in eine Körperhöhle zu schaffen. Dazu wird der Trokar mit Hilfe des Trokardorns durch die Körperdecke in den Körper gestochen. Anschließend wird der Trokardorn aus der Trokarhülse herausgezogen, während die Trokarhülse in dem nun geschaffenen Zugang zurückgelassen wird. Durch die Trokarhülse können dann abwechselnd Instrumente wie Endoskope, Zangen, Scheren, Nahtinstrumente und dergleichen zum Durchführen chirurgischer Maßnahmen in die Körperhöhle eingeführt werden.

Während einer Operation werden häufig mehrere Instrumente benötigt, die unterschiedliche Schaftdurchmesser aufweisen. Die Trokarhülse muss entsprechend für die Einführung dieser Instrumente ausgelegt sein.

Eine weitere Anforderung an eine Trokarhülse besteht darin, die Körperhöhle bei in den Körper eingeführter Trokarhülse und durch diese eingeführtem Instrument nach außen abzudichten. Dies ist insbesondere im Rahmen der Verwendung einer Trokarhülse in der Laparoskopie wichtig, bei der die Körperhöhle, hier der Bauchraum, mit einem Gas zur Aufweitung der Körperhöhle insuffliert wird (sog. Pneumoperitoneum).

Um bei durch die Trokarhülse eingeführtem Instrument eine Abdichtung der Körperhöhle nach außen zu bewerkstelligen, weisen derartige Trokarhülsen im Bereich des Trokarkopfes eine Dichtung auf, durch die das jeweilige Instrument hindurch geführt wird. Die Dichtung weist dazu ein elastisches Dichtelement auf, das sich umfänglich an den Schaft des eingeführten Instruments dichtend anschmiegt.

Da durch die Trokarhülse jedoch verschiedene Instrumente mit unterschiedlichen Schaftdurchmessern einführbar sein sollen, muss die in der Trokarhülse vorhandene Dichtung auch bei unterschiedlichen Schaftdurchmessern der eingeführten Instrumente jeweils eine Abdichtung gewährleisten. Es versteht sich, dass der Innendurchmesser des Dichtelements, der den Durchgang für das Instrument definiert, auf den kleinsten Schaftdurchmesser eines Instruments ausgelegt sein muss, um auch für dieses dünne Instrument eine Abdichtung zu gewährleisten.

Um aber auch das Einführen von Instrumenten mit größerem Schaftdurchmesser zu ermöglichen, muss das Dichtelement eine ausreichende radial elastische Dehnbarkeit besitzen. Zu diesem Zweck weisen die derzeit bekannten Dichtungen Dichtelemente auf, die sehr dünnwandig sind. Dies ist insbesondere bei einer aus dem Dokument DE 692 04 958 T2 bekannten Dichtung der Fall.

Diese bekannte Dichtung weist ein Dichtelement auf, das die Form eines Stundenglases besitzt. Das Dichtelement weist konvergierende und divergierende Seitenwände auf, die an ihrem Schnittpunkt den Durchgang für ein Instrument bilden. Die äußeren Enden der Dichtung sind durch Flanschabschnitte gebildet. In dem Dokument wird vorgeschlagen, den Innendurchmesser des Durchgangs durch Anlegen eines Vakuums oder eines negativen Druckes an die Außenseite des Dichtelements zu vergrößern. Die Realisierung einer Dichtung einer Trokarhülse, an der ein Vakuum angelegt werden kann, ist jedoch technisch aufwändig und daher nachteilig.

Ein weiterer Nachteil der oben genannten dünnwandigen Dichtelemente der bekannten Dichtungen besteht darin, dass die dünne Wand des Dichtelements schon bei Erstkontakt mit spitzen, scharfen oder hakenförmigen Instrumenten beschädigt werden kann.

Ein weiterer Nachteil besteht in einer schlechten Führung von Instrumenten mit kleineren Schaftdurchmessern in der Dichtung.

Außerdem geht die Abdichtwirkung bei den bekannten Dichtungen häufig verloren, wenn das durch die Trokarhülse eingeführte Instrument seitlich, d.h. quer zur Schaftrichtung bewegt oder verkippt wird. Durch den geringen seitlichen Halt der dünnwandigen Dichtelemente geht bei einer seitlichen Bewegung die Anlage zwischen dem Dichtelement und dem Instrumentenschaft auf einem Teilumfang des Instrumentenschafts verloren, wodurch jedoch eine ausreichende Abdichtung nicht mehr gewährleistet ist.

Eine aus dem Dokument DE 37 37 121 A1 bekannte Dichtung weist ebenfalls ein dünnwandiges Dichtelement auf, wobei das Dichtelement einen Hohlraum aufweist, der mit einem gasförmigen oder flüssigen Medium gefüllt wird, um den zu verschließenden Durchgang für das Instrument einzuengen, um die gewünschte Dichtwirkung zu erzielen. Diese bekannte Dichtung benötigt somit zur Erzielung der Dichtwirkung einen erhöhten Druck im Hohlraum des Dichtelements, der durch das Beaufschlagen des Hohlraums mittels einer Flüssigkeit oder eines Gases erzielt wird. Der Nachteil hierbei ist, dass der Trokar während eines chirurgischen Eingriffs mit einer Druckquelle über eine oder mehrere Leitungen verbunden werden muss, die während eines chirurgischen Eingriffs eine Behinderung des Arztes darstellen können. Außerdem ist der apparative Aufwand für die Druckbeaufschlagung des Dichtelements nachteiligerweise erhöht.

Aus der eingangs genannten WO 99/42152 A1 ist eine Dichtung für eine Trokarhülse bekannt, die gemäß einem in Fig. 8 dargestellten Ausführungsbeispiel zwei axial voneinander beabstandete elastische Dichtelemente in Form von Scheibendichtungen aufweist, die jeweils mittig einen Durchgang für ein Instrument definieren. Die beiden scheibenförmigen Dichtelemente sind an ihrem radial äußeren Ende einstückig miteinander verbunden und von einem dicht abgeschlossenen schlauchförmigen Hohlraum umgeben, der mit Luft gefüllt und dicht abgeschlossen ist. Der Hohlraum dient dazu, dass sich die Öffnungen in den scheibenförmigen Dichtelementen bei einer Schrägstellung des durchgeführten Instruments möglichst nicht so verzerren, dass die Ränder der Öffnungen mit dem Instrument teilweise außer Eingriff kommen. Der Hohlraum bzw. die Wandung des Hohlraums bildet demnach nicht den Durchgang für ein Instrument.

In einem in Fig. 9 in dem vorstehend genannten Dokument gezeigten Ausführungsbeispiel weist der Dichtungskörper nur ein scheibenförmiges Dichtelement auf, das wiederum von einem schlauchförmigen Hohlraum umgeben ist, der dicht abgeschlossen und mit einem Fluid gefüllt ist. Auch hier ist der Durchgang für ein Instrument mittig in dem scheibenförmigen Dichtelement angeordnet.

Aus US 5,788,676 ist eine Trokarhülse bekannt, die zwei Dichtungen aufweist, die jeweils wie oben beschrieben in Form eines Stundenglases ausgebildet sind. Im Bereich des Durchgangs für ein Instrument durch das jeweilige Dichtungselement ist jeweils ein kompressibles, schlauchförmiges, mit einem kompressiblen Material gefülltes Element angeordnet, das auf das eigentliche Dichtelement eine radial nach innen gerichtete Druckkraft ausübt. Der Durchgang für das Instrument wird durch das stundenglasförmige Dichtelement selbst gebildet.

Aus US 2005/0261661 A1 ist eine Dichtung für eine Trokarhülse bekannt, dessen Dichtungskörper aus einem elastomeren Vollmaterial gefertigt ist. Der Dichtungskörper dieser Dichtung ist in einem Gehäuse so aufgenommen, dass sich der Dichtungskörper bei Einführen eines Instruments radial nach außen ausdehnen kann.

Aus US 6,276,661 B1 ist eine Dichtung für eine Trokarhülse bekannt, dessen Dichtungskörper ein elastisches Dichtelement aufweist, das im Wesentlichen die Form eines Stundenglases aufweist. Zwischen zwei Ringflanschen dieses Dichtungskörpers wird ein starrer Haltering eingesetzt, wobei an dem Haltering eine Öffnung vorhanden ist, durch die die im Längsschnitt V-förmige Wand des Dichtelements mit einem Druck beaufschlagt werden kann, wodurch die V-förmige Wand des Dichtelements radial nach innen und in axialer Richtung gedehnt wird, um den Durchgang durch das Dichtelement zu verengen.

Weitere Dichtungen für medizinische Instrumente sind aus den Dokumenten FR 2 863 504 A1, EP 0 567 141 A2, EP 0 316 096 A2, US 5,685,854, US 5,782,817 und US 5,989,224 bekannt.

Der Erfindung liegt die Aufgabe zugrunde, eine Dichtung der eingangs genannten Art dahingehend weiterzubilden, dass sie eine ausreichende Abdichtung, insbesondere bei unterschiedlichen Durchmessern verwendeter Instrumente, ohne apparativen Mehraufwand gewährleistet.

Diese Aufgabe wird hinsichtlich der eingangs genannten Dichtung erfindungsgemäß dadurch gelöst, dass der Dichtungskörper in einem mittleren Bereich durch zumindest einen den Hohlraum außenseitig zumindest teilweise umgebenden Mittelstützring versteift ist.

Das Dichtelement der erfindungsgemäßen Dichtung weist demnach einen im Wesentlichen schlauchförmigen elastischen Hohlkörper auf, der ein Fluid enthält, das gasförmig oder flüssig sein kann. Die schlauchförmige Ausgestaltung des Dichtelements hat die Wirkung, dass beim Durchführen eines Instruments durch das Dichtelement hindurch der schlauchförmige Hohlraum radial komprimiert wird, wodurch der Anlagebereich des Dichtelements am Instrumentenschaft auch eine axiale Erstreckung aufweist. Das in dem Hohlraum enthaltende Fluid wird im Fall, dass der Hohlraum dicht abgeschlossen ist, wie es bspw. vorgesehen sein kann, bei der radialen Stauchung des Hohlraums komprimiert und bringt einen entsprechenden Gegendruck auf den Instrumentenschaft auf, der stets eine vollumfänglich dichtende Anlage des Dichtelements an den Instrumentenschaft gewährleistet. Je nach dem Schaftdurchmesser des eingeführten Instruments wird der Hohlraum mehr oder weniger komprimiert.

Die Ausgestaltung des Dichtelements als im Wesentlichen schlauchförmiger, ein Fluid enthaltender Hohlkörper, hat des Weiteren den Vorteil, dass bei einer radial gerichteten Bewegung des eingeführten Instruments das Fluid, das bei dieser Bewegung an der einen Stelle verdrängt wird, in dem gegenüberliegenden Bereich des Hohlraums einen erhöhten Druck auf den radial inneren Wandbereich des Dichtelements ausübt, so dass der radial innere Wandbereich an der Stelle erhöhten Drucks sogar radial nach innen gedehnt wird, wodurch bei einer seitlichen Bewegung des Instrumentenschafts stets eine vollumfängliche dichtende Anlage des Dichtelements am Instrumentenschaft gewährleistet bleibt. Das Gleiche gilt bei einer Verkippung des Instruments gegenüber dem Dichtelement.

Dadurch, dass der radial äußere Wandbereich des Dichtelements eine Wandstärke aufweist, die größer ist als die Wandstärke des radial inneren Wandbereichs, übt der radial äußere Wandbereich bei einem durch das Dichtelement durchgeführten Instrument eine radial nach innen gerichtete Gegenkraft aus, so dass eine ausreichende Abdichtung zwischen dem eingeführten Instrument und der radial inneren Dichtungswand erreicht wird, ohne dass der Hohlraum der Dichtung mit einem Gas oder einer Flüssigkeit zur Erzielung eines Überdrucks gefüllt werden muss, um diese Dichtwirkung zu erzielen. Die erfindungsgemäße Dichtung kommt somit ohne einen Anschluss an eine Luft- oder Flüssigkeitsquelle aus, was einerseits den apparativen Aufwand gering hält, und andererseits werden Hindernisse wie Schläuche oder Leitungen im Operationsfeld vermieden.

Der Hohlraum kann in radialer Richtung, d.h. quer zur Längsmittelachse des Dichtungskörpers vorzugsweise einen maximalen Innendurchmesser aufweisen, der dem größten Durchmesser verwendeter Instrumente entspricht.

Der Dichtungskörper ist in einem mittleren Bereich durch zumindest einen den Hohlraum außenseitig zumindest teilweise umgebenden Mittelstützring versteift.

Hierbei ist von Vorteil, dass die beim Durchführen eines Instruments durch das Dichtelement entstehenden radial nach außen wirkenden Kräfte nicht zu einer radialen Dehnung des gesamten Dichtungskörpers führen, sondern nur das Dichtelement radial gedehnt wird. Ein radiales Ausweichen des Dichtelements nach außen wird somit vermieden, was die Abdichtung gegenüber dem Instrument weiter verbessert.

In einer bevorzugten Ausgestaltung ist das Fluid ein Gas, insbesondere Luft.

Die Verwendung eines Gases als Fluid hat den Vorteil, dass ein Gas kompressibel ist, so dass der Hohlraum die elastischen Eigenschaften in der Art einer Gasfeder besitzt. Als Gas wird vorzugsweise Luft verwendet, wobei ein Lufteinschluss in den Hohlraum bereits bei der Fertigung der Dichtung mit einfachen Maßnahmen bewerkstelligt werden kann. Insbesondere gemäß einer später noch zu beschreibenden Ausgestaltung kann der Hohlraum jedoch auch nicht in sich luftdicht abgeschlossen sein, sondern eine gewisse Undichtigkeit aufweisen, die ein Entweichen des Fluids bei größeren Schaftdurchmessern ermöglicht. Bei Verwendung von Luft als Fluid kann sich der Hohlraum beim Entspannen oder bei Verwendung eines Instruments mit kleinerem Durchmesser dann wieder selbsttätig mit Luft aus der Umgebung füllen, ohne dass die Dichtung mit einem Gasreservoir verbunden sein muss.

In einer weiteren bevorzugten Ausgestaltung ist das Fluid eine Flüssigkeit, vorzugsweise ein Öl.

Eine Flüssigkeit ist gegenüber einem Gas weniger kompressibel bzw. inkompressibel, kann jedoch bei kleineren Schaftdurchmessern einen höheren Gegendruck zum festen Andrücken des radial inneren Wandbereichs an den Schaft des eingeführten Instruments ermöglichen. Für größere Schaftdurchmesser muss jedoch das Fluid aus dem Hohlraum entweichen können, wozu ein entsprechendes Ausgleichsreservoir vorzusehen wäre.

In einer weiteren bevorzugten Ausgestaltung steht das Fluid im entspannten Zustand des Dichtelements unter Umgebungsdruck.

Diese Maßnahme ist insbesondere mit der zuvor genannten Ausgestaltung, wonach das Fluid Luft ist, vorteilhaft, da keine aufwändigen Maßnahmen getroffen werden müssen, um im entspannten Zustand des Dichtelements in dem Hohlraum einen gegenüber dem Umgebungsdruck erhöhten Druck bereitzustellen.

In einer weiteren bevorzugten Ausgestaltung ist der radial innere Wandbereich des Hohlraums im entspannten Zustand des Dichtelements von der Längsmittelachse aus gesehen, konvex gewölbt.

Diese Ausgestaltung hat den Vorteil, dass das Einführen von Instrumenten durch das Dichtelement hindurch vereinfacht ist, weil sich der Durchgang für das Instrument, in Einführrichtung gesehen, trichterförmig verjüngt. Die Gefahr, dass das Instrument den radial inneren Wandbereich des Dichtelements beschädigt, ist somit verringert. Das Gleiche gilt beim Herausziehen des Instruments aus der Trokarhülse, da auch vom zweiten Ende des Dichtelements aus gesehen, der Durchgang sich trichterförmig verjüngt.

In einer weiteren bevorzugten Ausgestaltung ist der Hohlraum in einem Schnitt entlang der Längsmittelachse im Wesentlichen O-förmig oder oval.

Im Falle eines ovalförmigen Hohlraums erstreckt sich die größere Ausdehnung des Hohlraums vorzugsweise in Richtung der Längsmittelachse. Der Vorteil der O-förmigen oder ovalen Ausgestaltung des Hohlraums besteht unter anderem darin, dass das Dichtelement beim Einführen eines Instruments in axialer Richtung nicht oder nur geringfügig ausweicht und dem eingeführten Instrument dadurch eine bessere Lagestabilität verleiht.

In einer weiteren bevorzugten Ausgestaltung weist zumindest der radial innere Wandbereich des Dichtelements eine wesentlich gleichmäßige Wandstärke auf.

Da der radial innere Wandbereich des Dichtelements die Anlagefläche des Dichtelements an einem eingeführten Instrument bildet, führt die im Wesentlichen gleichmäßige Wandstärke in diesem Bereich zu einer gleichmäßigen Verformung des Dichtelements und somit einer gleichmäßigen und gut abdichtenden Anlage des Dichtelements am Instrumentenschaft. Gegenüber den bekannten Dichtungen ist es jedoch nicht erforderlich, die Wandstärke des Dichtelements sehr dünn zu halten, weil die radiale Dehnbarkeit des Dichtelements durch den Hohlraum mitbestimmt wird.

In einer weiteren bevorzugten Ausgestaltung weist der radial innere Wandbereich des Hohlraums eine radial noch weiter nach innen gerichtete wulstringförmige Verdickung auf.

Die wulstringförmige Verdickung bildet vorteilhafterweise eine Verstärkung des Dichtelements im mittigen Bereich des radial inneren Wandbereichs des Dichtelements, die die Abdichtung verbessert.

In einer weiteren bevorzugten Ausgestaltung ist der Dichtungskörper im Bereich zumindest eines der axialen Enden durch zumindest einen sich zumindest teilumfänglich erstreckenden Stützring versteift.

Das Vorsehen eines Stützrings an zumindest einem der Enden des Dichtungskörpers hat den Vorteil, dass der Dichtungskörper insgesamt aus einem relativ weichen elastomeren Material gefertigt werden kann, einschließlich des Dichtelements, während der zumindest eine Stützring dem Dichtungskörper die notwendige Formstabilität verleiht.

Bevorzugt ist es entsprechend, wenn der Dichtungskörper im Bereich beider axialen Enden jeweils zumindest einen sich zumindest teilumfänglich erstreckenden Stützring aufweist.

Des Weiteren ist es bevorzugt, wenn der zumindest eine Stützring in einer Ausnehmung des Dichtungskörpers aufgenommen ist, wobei ein Außendurchmesser des zumindest einen Stützrings größer ist als ein radialer äußerer Durchmesser der Ausnehmung.

Diese Maßnahme bewirkt, dass der Stützring den Dichtungskörper an dem zumindest einen Ende, vorzugsweise an beiden Enden radial aufweitet, wodurch der gesamte Dichtungskörper tailliert ist. Dies hat den Vorteil, dass die Abdichtung des Dichtungskörpers als solchem zum Gehäuse des Trokarkopfes, in dem die Dichtung angeordnet ist, verbessert ist. Die nur an den beiden Enden des Dichtungskörpers vorhandene radiale Aufweitung erleichtert auch das Auswechseln der Dichtung, da der Dichtungskörper nur an seinen axialen Enden mit innigem Kontakt an der Innenwand des Gehäuses des Trokarkopfes anliegt.

In einer weiteren bevorzugten Ausgestaltung weist der Hohlraum an seinem radial äußeren Umfang einen sich zumindest teilumfänglich erstreckenden Ringspalt auf, wobei der zumindest eine Mittelstützring den Ringspalt verschließt.

Diese Maßnahme hat nun den Vorteil, dass die Fertigung der Dichtung im Hinblick auf den vorzusehenden Hohlraum vereinfacht ist, da dieser zunächst radial außenseitig über den Ringspalt als offener Hohlraum gefertigt werden kann, wobei mittels des zumindest einen Mittelstützrings dieser Ringspalt dann verschlossen wird. Der Mittelstützring kann dabei den Hohlraum dicht verschließen, indem der Mittelstützring bspw. eingeklebt wird, oder der Mittelstützring wird lediglich in den Ringspalt eingelegt, und durch die Montage der Dichtung in den Trokarkopf wird die Dichtung dann geringfügig axial komprimiert, wodurch der Ringspalt durch Andrücken gegen den Mittelstützring verschlossen wird.

In einer weiteren bevorzugten Ausgestaltung kommuniziert der Hohlraum über zumindest eine Öffnung kleinen Öffnungsquerschnitts mit dem Außenraum, die vorzugsweise in dem zumindest einen Mittelstützring vorgesehen ist.

Bei dieser Ausgestaltung ist der Hohlraum des Dichtelements nicht vollkommen in sich dicht, sondern das darin enthaltene Fluid kann aus dem Hohlraum über die zumindest eine Öffnung verdrängt werden und beim Entspannen wieder in den Hohlraum gelangen. Diese Maßnahme hat insbesondere den Vorteil, dass Instrumente mit großem Schaftdurchmesser durch die Dichtung hindurchgeführt werden, die eine starke Komprimierung des Hohlraums bewirken. Bei Instrumenten mit derartig großen Schaftdurchmessern ist es auch nicht zwingend erforderlich, dass das in dem Hohlraum enthaltene Fluid die Andrückkraft zum Andrücken des radial inneren Wandbereichs des Dichtelements an den Instrumentenschaft bereitstellt, da bei derartig großen Schaftdurchmessern diese Andrückkraft bereits von der elastischen Wand des Hohlraums aufgebracht werden kann.

In einer weiteren bevorzugten Ausgestaltung ist das Dichtelement aus einem weichen elastomeren Material, vorzugsweise aus Silikon, gefertigt.

Die Verwendung eines weichen elastomeren Materials, insbesondere Silikon hat den Vorteil, dass die Reibung zwischen dem Instrumentenschaft und dem Dichtelement beim Einführen des Instruments durch den Trokar gering ist. Das Dichtelement kann insbesondere mit dem übrigen Dichtungskörper, mit Ausnahme der zuvor erwähnten Stützringe, einstückig gefertigt sein.

Die Dichtung ist vorzugsweise autoklavierbar.

Die zuvor genannten Stützringe und ggf. der Mittelstützring weisen vorzugsweise das Material PEEK und/oder ein Metall auf.

PEEK ist gegenüber Silikon steifer und eignet sich daher zur Versteifung des Dichtungskörpers wie oben beschrieben und ist im Übrigen auch autoklavierbar. Für eine besonders hohe Steifigkeit können die Stützringe bzw. der Mittelstützring auch aus einem Metall gefertigt sein oder durch eine Metallarmierung verstärkt sein.

In einer weiteren bevorzugten Ausgestaltung weist der Dichtungskörper ein Ventil, insbesondere ein Lippenventil, auf.

Üblicherweise weisen Trokarhülsen neben einer Dichtung zum radialen Abdichten gegen den Instrumentenschaft auch ein Ventil auf, das eine axiale Abdichtung der Trokarhülse bewirkt, wenn kein Instrument durch die Trokarhülse eingeführt ist, bspw. bei einem Instrumentenwechsel. Üblicherweise stellen die Ventile separate Bauteile dar. Die vorstehend genannte Ausgestaltung hat demgegenüber den Vorteil, dass das Ventil mit dem Dichtungskörper als Baueinheit gefertigt werden kann, was insbesondere auch den Zusammenbau der Trokarhülse vereinfacht, da weniger Einzelteile vorhanden sind.

Dabei ist es weiterhin bevorzugt, wenn das Ventil einstückig mit dem radial inneren Wandbereich des Dichtelements ausgebildet ist.

Hierbei ist nun von Vorteil, dass das Ventil, insbesondere ein Lippenventil, in das Dichtelement selbst integriert ist, was die Gestehungskosten einer Trokarhülse vorteilhafterweise verringert.

Die Erfindung betrifft des Weiteren eine Trokarhülse, mit einem Rohrschaft und einem Trokarkopf, der am proximalen Ende des Rohrschafts angeordnet ist, sowie mit einer im Trokarkopf angeordneten Dichtung nach einer oder mehreren der vorstehend genannten Ausgestaltungen.

Weitere Vorteile und Merkmale gehen aus der nachfolgenden Beschreibung und der beigefügten Zeichnung hervor.

Es versteht sich, dass die vorstehend genannten und nachstehend zu erläuternden Merkmale nicht an die jeweils angegebenen Kombinationen, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in Bezug auf diese hiernach näher beschrieben. Es zeigen:
- Fig. 1: eine Seitenansicht einer Trokarhülse;
- Fig. 2: einen Längsmittelschnitt durch einen Trokarkopf der Trokarhülse in Fig. 1 in vergrößertem Maßstab;
- Figuren 3a) bis c): eine Dichtung der Trokarhülse in Fig. 1 in Alleinstellung, wobei Fig. 3a) die Dichtung in einem Längsmittelschnitt entlang der Schnittlinie IIIa-IIIa in Fig. 3c) in vergrößertem Maßstab zeigt, während Fig. 3b) die Dichtung in Seitenansicht und Fig. 3c) die Dichtung in Draufsicht zeigt;
- Figuren 4a) bis c): die Dichtung in Fig. 3 in Figuren 3a) bis 3c) entsprechenden Ansichten, wobei die Dichtung mit einem ausschnittsweise dargestellten und durch die Dichtung hindurchgehenden Instrumentenschaft gezeigt ist;
- Figuren 5a) bis c): eine Dichtung zur Verwendung in dem Trokar in Fig. 1 gemäß einem weiteren Ausführungsbeispiel, wobei Fig. 5a) die Dichtung in Draufsicht, Fig. 5b) die Dichtung in einem Längsmittelschnitt entlang der Linie Vb-Vb in Fig. 5a) und Fig. 5c) die Dichtung in einem Längsmittelschnitt entlang der Schnittlinie Vc-Vc in Fig. 5a) zeigt;
- Fig. 6: einen Längsmittelschnitt durch einen Trokarkopf in Abwandlung des Ausführungsbeispieles in Fig. 2; und
- Fig. 7: einen vergrößerten Ausschnitt B in Fig. 6.

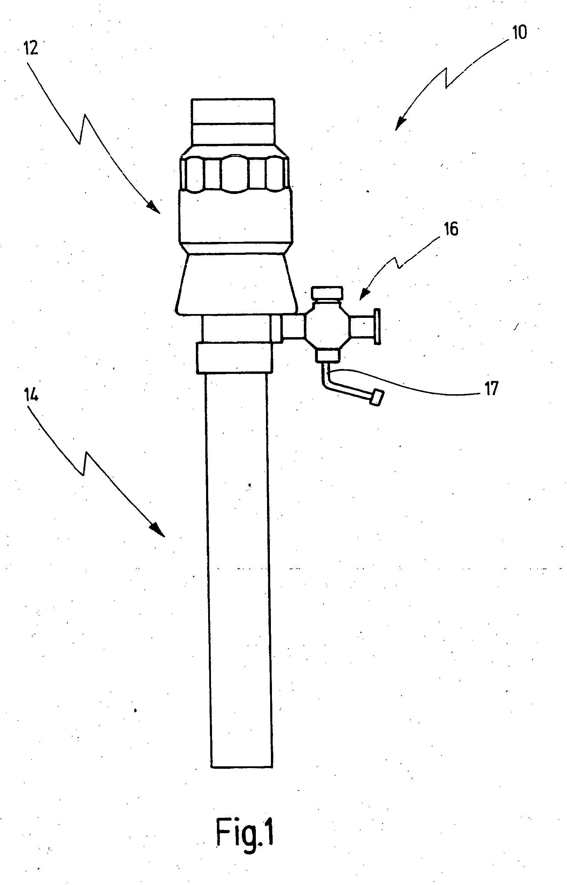

In Fig. 1 ist eine mit dem allgemeinen Bezugszeichen 10 versehene Trokarhülse dargestellt. Die Trokarhülse 10 wird in minimal-invasiven chirurgischen Verfahren zum Einführen von Instrumenten in eine Körperhöhle verwendet. Zusammen mit einem nicht dargestellten Trokardorn bildet die Trokarhülse 10 einen Trokar.

Die Trokarhülse 10 weist einen Trokarkopf 12 sowie einen Rohrschaft 14 auf, der mit dem Trokarkopf 12 lösbar verbunden ist. Am Trokarkopf 12 ist ferner ein Anschluss 16, bspw. zum Anschließen eines Schlauches zum Zuführen eines Insufflationsgases in die Körperhöhle angeordnet. Zum Öffnen und Schließen ist der Anschluss 16 mit einem Hahn 17 versehen.

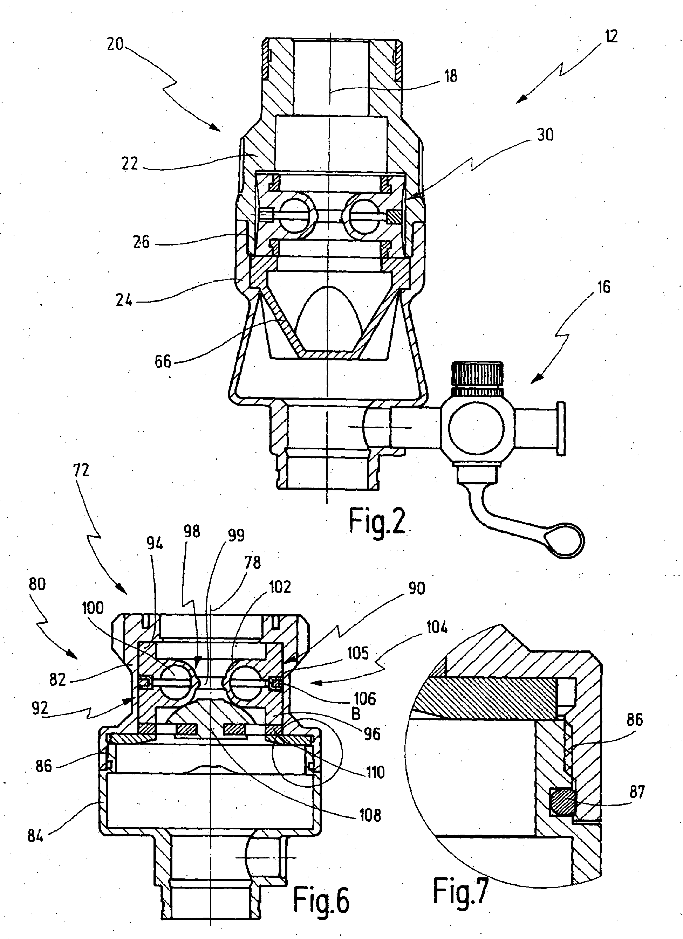

In Fig. 2 ist der Trokarkopf 12 der Trokarhülse 10 in Alleinstellung in einem Längsmittelschnitt entlang einer Längsmittelachse 18 und gegenüber Fig. 1 vergrößert dargestellt.

Der Trokarkopf 12 weist ein Gehäuse 20 auf, das ein erstes Gehäuseteil 22 und ein zweites Gehäuseteil 24 aufweist, wobei die beiden Gehäuseteile 22 und 24 über eine Verschraubung 26 lösbar miteinander verbunden sind.

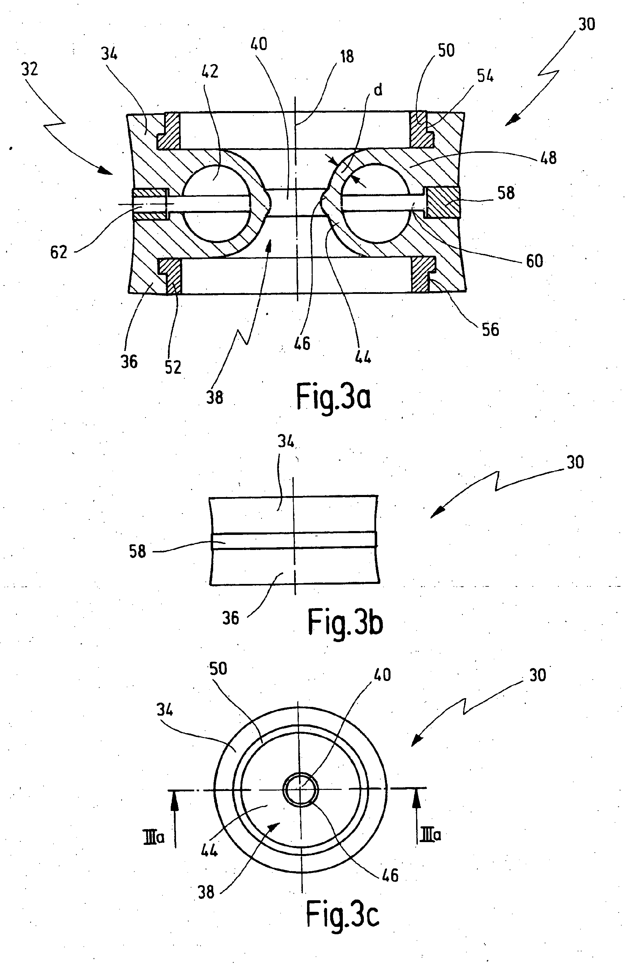

In dem ersten Gehäuseteil 22 ist eine Dichtung 30 angeordnet, die hiernach mit Bezug auf Figuren 3a) bis c) beschrieben wird.

Die Dichtung 30 weist allgemein einen Dichtungskörper 32 auf, der in Richtung der Längsmittelachse 18 des Dichtungskörpers 32 ein erstes axiales Ende 34 und ein zweites axiales Ende 36 aufweist. Der Dichtungskörper 32 ist gemäß der Draufsicht in Fig. 3c) kreisförmig ausgebildet.

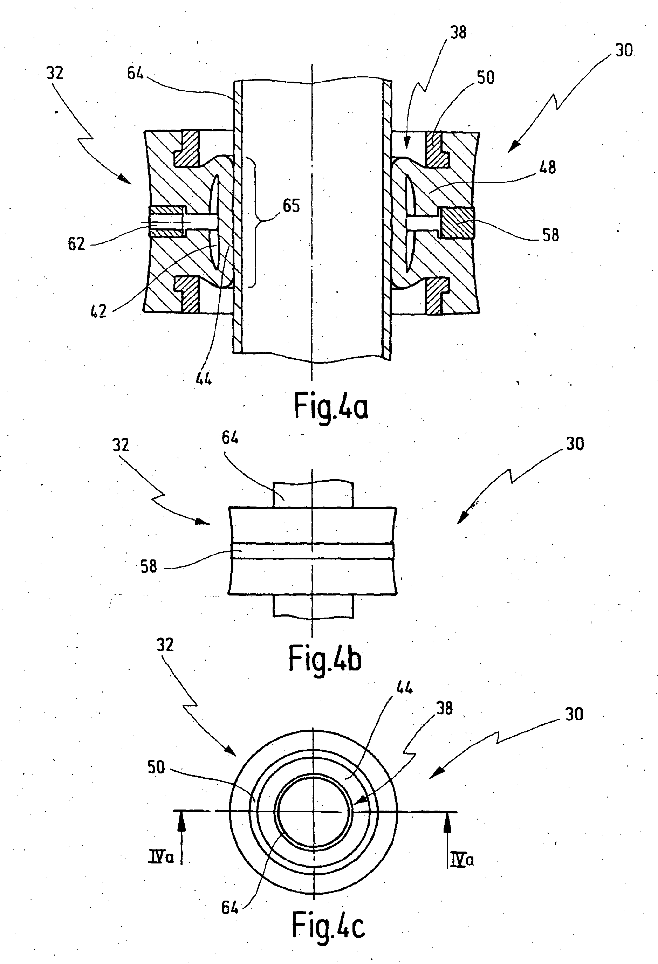

Zwischen dem ersten axialen Ende 34 und dem zweiten axialen Ende 36 weist der Dichtungskörper 32 ein elastisches Dichtelement 38 auf. In einem bezüglich der Längsmittelachse 18 radial inneren Bereich definiert das Dichtelement 38 einen Durchgang 40 für ein einzuführendes Instrument, wie später noch mit Bezug auf Fig. 4a) bis c) beschrieben wird.

Das Dichtelement 38 weist einen im Wesentlichen schlauchförmigen Hohlraum 42 auf, der ein Fluid in Form eines Gases, insbesondere Luft enthält. Ein radial innerer Wandbereich 44 des Dichtelements 38, der den Hohlraum 42 auf der radial innenliegenden Seite, d.h. auf der der Längsmittelachse 18 zugewandten Seite begrenzt, umgibt den Durchgang 40 vollumfänglich und ringförmig. Das Dichtelement 38 ist demnach als Hohlkörper ausgebildet.

Wie aus Fig. 3a) hervorgeht, weist der vollumfänglich ausgebildete radial innere Wandbereich 44 auch eine signifikante axiale Erstreckung in Richtung der Längsmittelachse 18 auf, die in dem gezeigten Ausführungsbeispiel sogar etwa zwei Drittel der gesamten axialen Erstreckung des Dichtungskörpers 32 beträgt. Dieses Maß ist jedoch nur beispielhaft zu verstehen.

Figuren 3a) bis c) zeigen die Dichtung 30 im entspannten Zustand des Dichtelements 38, d.h. in einem Zustand, in dem kein Instrument durch die Dichtung 30 hindurchgeführt ist. In diesem Zustand nimmt der Hohlraum 42 in dem gezeigten Schnitt gemäß Fig. 3a) eine ovale Form ein, wobei die längere Abmessung des Ovals in Richtung der Längsmittelachse 18 weist. Der radial innere Wandbereich 44 des Dichtelements 38 weist eine im Wesentlichen gleichmäßige Wandstärke d auf. Die Wandstärke d liegt bspw. in einem Bereich von etwa 0,8 bis etwa 1,3 mm.

Von der Längsmittelachse 18 aus gesehen ist die radial innere Wand 44 konvex gewölbt. Der radial innere Wandbereich 44 ist des Weiteren mit einer radial nach innen gerichteten vorspringenden wulstringförmigen Verdickung 46 versehen.

Ein radial äußerer Wandbereich 48 des Dichtelements 38 geht in die Enden 34 und 36 des Dichtungskörpers 32 über. Insbesondere ist das Dichtelement 38 zusammen mit den Enden 34 und 36 des Dichtungskörpers 32 einstückig ausgebildet. Der radial äußere Wandbereich 48 weist eine größere Wandstärke auf als der radial innere Wandbereich 44, so dass der radial äußere Wandbereich 48 formstabiler ist als der radial innere Wandbereich 44.

Das Dichtelement 38 mit dem radial inneren Wandbereich 44 und dem radial äußeren Wandbereich 48 sowie den übrigen Abschnitten im Bereich der Enden 34 und 36 des Dichtungskörpers 32 sind aus einem weichen elastomeren Material gefertigt, bspw. aus Silikon.

Der Dichtungskörper 32 ist im Bereich der beiden axialen Enden 34 und 36 mit jeweils einem Stützring 50 und 52 versteift. Die Stützringe 50 und 52 sind vorzugsweise aus einem steifen Material, insbesondere aus PEEK und/oder aus Metall gefertigt. Die Stützringe 50 und 52 erstrecken sich in dem gezeigten Ausführungsbeispiel vollumfänglich am Dichtkörper 32, wie in Fig. 3c) für den Stützring 50 dargestellt ist.

Der Stützring 50 ist in einer Ausnehmung des Dichtungskörpers 32 aufgenommen, wobei ein Außendurchmesser des Stützrings 50 größer ist als ein radial äußerer Durchmesser der Ausnehmung 54, wodurch der Stützring 50 den Dichtungskörper 32 im Bereich des Endes 34 radial nach außen aufweitet.

Der Stützring 52 ist in einer entsprechenden Ausnehmung 56 des Dichtungskörpers 32 im Bereich des zweiten Endes 36 aufgenommen, wobei auch hier der Stützring 52 relativ zur Ausnehmung 56 so bemessen ist, dass der Stützring 52 den Dichtungskörper 32 auch im Bereich des zweiten Endes 36 radial nach außen aufweitet. Insgesamt ergibt sich hierdurch eine taillierte Außenkontur des Dichtungskörpers 32, wie aus Figuren 3a) und 3b) hervorgeht.

In einem mittleren Bereich zwischen den Enden 34 und 36 ist der Dichtungskörper 32 ferner durch einen Mittelstützring 58 versteift. Der Mittelstützring 58 ist axial auf Höhe des Hohlraums 42 des Dichtelements 38 angeordnet und umgibt den Hohlraum 42 vollumfänglich.

Der Mittelstützring 58 verschließt dabei einen Ringspalt 60, der im radial äußeren Wandbereich 48 des Dichtelements 38 vollumfänglich ausgebildet ist. Der Hohlraum 42 ist somit radial außenseitig offen gebildet, was die Fertigung des Dichtungskörpers 32 mit dem Dichtelement 38 vereinfacht. Der Mittelstützring 58 versteift nicht nur den Dichtungskörper 32 im mittleren Bereich zwischen den Enden 34 und 36, sondern verschließt gleichzeitig den Ringspalt 60. Der Mittelstützring 58 verschließt den Hohlraum 42 jedoch nicht vollständig, sondern lässt eine wenn auch geringe Kommunikation des Hohlraums 42 mit dem Außenraum des Hohlraums 42 zu, indem in dem Mittelstützring 58 eine Öffnung 62 kleinen Öffnungsquerschnitts in Form einer dünnen Bohrung vorhanden ist.

Im entspannten Zustand des Dichtelements 38 gemäß Fig. 3a) steht das Fluid, hier Luft, unter Umgebungsdruck, der sich aufgrund der Öffnung 62 stets von selbst in dem Hohlraum 42 einstellt.

Figuren 4a) bis c) zeigen die Dichtung 30 in einem Zustand, in dem ein Instrument, von dem in Figuren 4a) bis c) ein Instrumentenschaft 64 ausschnittsweise dargestellt ist, durch die Dichtung 30 hindurchgeht. In diesem Fall ist der radial innere Wandbereich 44 des Dichtelements 38 unter Kompression des Hohlraums 42 derart verformt, dass der radial innere Wandbereich 44 über einen sich axial weit erstreckenden Bereich 65 innig an den Instrumentenschaft 64 anschmiegt. Dies erhöht die Lagestabilität des Instruments in der Dichtung 30. Die in dem Hohlraum 42 enthaltene Luft ist dabei teilweise aus der Öffnung 62 verdrängt worden. Wie des Weiteren aus Fig. 4a) und 4b) hervorgeht, ist der übrige Dichtungskörper 32 bzw. der radial äußere Wandbereich 48 des Dichtelements 38 nicht oder im Wesentlichen nicht verformt, sondern lediglich der radial innere Wandbereich 44, was durch den Mittelstützring 58 gewährleistet wird. Der Mittelstützring 58 vermeidet somit ein Ausweichen des Dichtelements 38 radial nach außen, wodurch eine ausreichende Andrückkraft des radial inneren Wandbereichs 44 an dem Instrumentenschaft zur Erzielung einer hohen Abdichtung gewährleistet wird.

Nach Herausziehen des Instrumentenschafts 64 aus dem Durchgang 40 im Dichtelement 38 nimmt das Dichtelement 38 und insbesondere der Hohlraum 42 wieder seine Gestalt gemäß Fig. 3a) ein, wobei sich der Hohlraum 42 durch die Öffnung 62 hindurch wieder mit Luft füllt.

Wie Fig. 2 zeigt, hat die taillierte Ausgestaltung der Dichtung 30 im Bereich der Enden 34 und 36 den Vorteil, dass der Dichtungskörper 32 der Dichtung 30 im Bereich der Enden 34 und 36 gegen das Gehäuse 20, d.h. das erste Gehäuseteil 22 gut abgedichtet ist.

Während oben beschrieben wurde, dass der Hohlraum 42 des Dichtelements 38 nicht in sich vollkommen dicht geschlossen ist, kann ebenso vorgesehen sein, den Hohlraum 42 vollkommen dicht auszugestalten, wobei dann die Öffnung 62 in dem Mittelstützring 58 oder auch der Ringspalt 60 in dem Dichtungskörper 32 entfällt.

Des Weiteren kann der Hohlraum 42 anstelle mit Luft oder einem anderen Gas mit einer Flüssigkeit wie bspw. einem Öl gefüllt sein.

Weiter mit Bezug auf Fig. 2 ist in dem Gehäuse 20 des Trokarkopfes 12 ein Ventil 66 angeordnet, das die Trokarhülse 10 abdichtet, wenn kein Instrument wie in Figuren 4a) bis c) eingeführt ist. Beim Einführen des Instrumentenschafts 64 wird das Ventil 66 aufgestoßen. In dem in Fig. 2 gezeigten Ausführungsbeispiel ist das Ventil 66 als Lippenventil ausgestaltet.

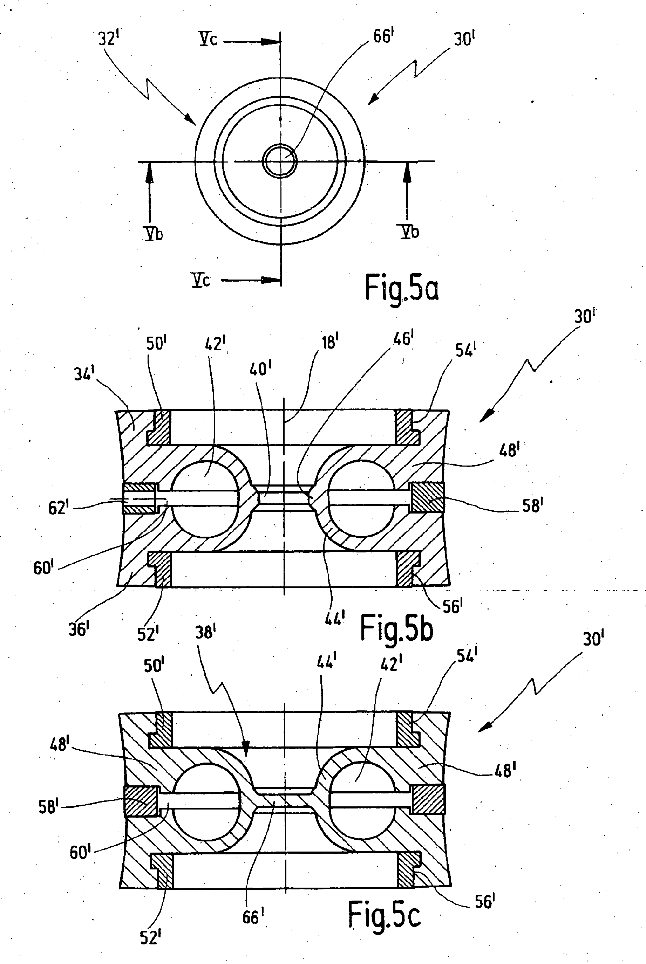

Ein solches Lippenventil kann jedoch auch in die Dichtung 30 integriert sein, wie bei dem Ausführungsbeispiel gemäß Figuren 5a) bis c) gezeigt ist.

Die dort dargestellte Dichtung 30' unterscheidet sich von der Dichtung 30 lediglich dadurch, dass im Durchgang 40' ein Ventil 66' in Form eines Lippenventils angeordnet ist, das einstückig mit dem radial inneren Wandbereich 44' des Dichtelements 38' ausgebildet ist. Bei Verwendung der Dichtung 30' anstelle der Dichtung 30 in Fig. 2 kann somit auf das Ventil 66 verzichtet werden, wodurch einerseits eine axial kürzere Bauweise des Trokarkopfes 12 erreicht wird, und andererseits Teile eingespart werden, wodurch die Montage und die Gestehungskosten des Trokarkopfes 12 verringert werden.

Die übrige Ausgestaltung der Dichtung 30' entspricht der Dichtung 30, so dass auf die obige Beschreibung verwiesen werden kann. In Figuren 5a) bis c) wurden identische oder ähnliche Merkmale mit den gleichen Bezugszeichen ergänzt durch einen hochgestellten Strich, versehen.

In Figuren 6 und 7 ist ein weiteres Ausführungsbeispiel eines Trokarkopfes 72 dargestellt, der anstelle des Trokarkopfes 12 bei der Trokarhülse 10 in Fig. 1 mit dem Rohrschaft 14 verbunden werden kann. Der Trokarkopf 72 weist ein Gehäuse 80 auf, das aus einem ersten Gehäuseteil 82 und einem zweiten Gehäuseteil 84 gebildet ist. Das erste Gehäuseteil 82 und das zweite Gehäuseteil 84 sind über eine Verschraubung 86 miteinander verbunden. Eine O-Ringdichtung 87 (Fig. 7) sorgt für eine zusätzliche Abdichtung der beiden Gehäuseteile 82 und 84 gegeneinander. Eine Längsmittelachse des Trokarkopfes 72 ist mit dem Bezugszeichen 78 versehen.

In dem ersten Gehäuseteil 82 ist eine Dichtung 90 angeordnet, die einen Dichtungskörper 92 aufweist, der ein erstes axiales Ende 94 und ein zweites axiales Ende 96 aufweist. Zwischen den Enden 94, 96 weist der Dichtungskörper 92 ein Dichtelement 98 mit Durchgang 99 auf, das gleich zu dem Dichtelement 38 der Dichtung 30 ausgebildet ist und insbesondere einen Hohlraum 100 mit einem radial inneren Wandbereich 102 wie oben beschrieben aufweist.

Im Unterschied zu der Dichtung 30 ist der Dichtungskörper 92 im Bereich der Enden 94, 96 nicht mit Stützringen versehen, sondern weist lediglich einen Mittelstützring 104 auf, der hier zweiteilig aus einem Kunststoffring 105, bspw. aus PEEK, gebildet ist, in den ein weiterer Ring 106, bspw. aus Metall, eingelegt ist.

Im Unterschied zu der Dichtung 30 ist die Außenkontur der Dichtung 90 nicht tailliert.

Mit der Dichtung 90 ist ferner ein Ventil 108 verbunden, das hier als Klappenventil ausgebildet ist. Eine Halterung 110 des Klappenventils kann dabei fest mit dem Dichtungskörper 92 der Dichtung 90 verbunden oder gar in diesen integriert sein.

In der Außenseite des Dichtungskörpers 92 können Aufnahmen (nicht dargestellt) ausgebildet sein, die sich axial von dem Ventil 108 bis zum Mittelstützring 104 erstrecken, um das Ventil 108 an dem Dichtungskörper 92 zu befestigen.

## Patentansprüche

1. Dichtung für eine Trokarhülse (12), mit einem Dichtungskörper (32; 32'; 92), der in Richtung einer Längsmittelachse (18, 18'; 78) des Dichtungskörpers (32; 32'; 92) ein erstes axiales Ende (34; 34'; 94) und ein zweites axiales Ende (36; 36'; 96) und zwischen dem ersten und zweiten Ende (34, 36; 34', 36'; 94, 96) ein elastisches Dichtelement (38; 38'; 98) aufweist, das in einem bezüglich der Längsmittelachse (18, 18'; 78) radial inneren Bereich einen Durchgang (40; 40'; 99) für ein Instrument definiert, wobei das Dichtelement (38; 38'; 98) einen im Wesentlichen schlauchförmigen Hohlraum (42; 42'; 100) aufweist, dessen radial innerer Wandbereich (44; 44'; 102) den Durchgang (40; 40'; 99) ringförmig umgibt, und wobei der Hohlraum (42; 42'; 100) ein Fluid enthält, wobei das Dichtelement (38; 38'; 98) einen radial äußeren Wandbereich (48; 48') radial außerhalb des Hohlraums (42; 42'; 100) aufweist, dessen Wandstärke größer ist als die Wandstärke des radial inneren Wandbereichs (44; 44'; 102), **dadurch gekennzeichnet, dass** der Dichtungskörper (32; 32'; 92) in einem mittleren Bereich durch zumindest einen den Hohlraum (42; 42'; 100) außenseitig zumindest teilweise umgebenden Mittelstützring (58; 58'; 104) versteift ist.

2. Dichtung nach Anspruch 1, **dadurch gekennzeichnet, dass** das Fluid ein Gas, insbesondere Luft ist.

3. Dichtung nach Anspruch 1, **dadurch gekennzeichnet, dass** das Fluid eine Flüssigkeit, vorzugsweise ein Öl ist.

4. Dichtung nach Anspruch 2, **dadurch gekennzeichnet, dass** das Fluid Luft ist und im entspannten Zustand des Dichtelements (38; 38'; 98) unter Umgebungsdruck steht.

5. Dichtung nach einem der Ansprüche 1 bis 4, **dadurch gekennzeichnet, dass** der radial innere Wandbereich (44; 44'; 102) des Dichtelements (38; 38'; 98) im entspannten Zustand desselben von der Längsmittelachse (18; 18'; 78) aus gesehen konvex gewölbt ist.

6. Dichtung nach einem der Ansprüche 1 bis 5, **dadurch gekennzeichnet, dass** der Hohlraum (42; 42'; 100) in einem Schnitt entlang der Längsmittelachse (18; 18'; 78) im Wesentlichen O-förmig oder oval ist.

7. Dichtung nach einem der Ansprüche 1 bis 6, **dadurch gekennzeichnet, dass** zumindest der radial innere Wandbereich (44; 44'; 102) des Dichtelements (38; 38'; 98) eine im Wesentlichen gleichmäßige Wandstärke aufweist.

8. Dichtung nach einem der Ansprüche 1 bis 7, **dadurch gekennzeichnet, dass** der radial innere Wandbereich (44; 44'; 102) des Dichtelements (38; 38'; 98) eine radial noch weiter nach innen gerichtete wulstringförmige Verdickung (46; 46') aufweist.

9. Dichtung nach einem der Ansprüche 1 bis 8, **dadurch gekennzeichnet, dass** der Dichtungskörper (32; 32') im Bereich zumindest eines der axialen Enden (34, 36; 34', 36') durch zumindest einen sich zumindest teilumfänglich erstreckenden Stützring (50, 52; 50', 52') versteift ist.

10. Dichtung nach Anspruch 9, **dadurch gekennzeichnet, dass** der zumindest eine Stützring (50, 52; 50', 52') in einer Ausnehmung (54, 56; 54', 56') des Dichtungskörpers (32; 32') aufgenommen ist, wobei ein Außendurchmesser des zumindest einen Stützrings (50, 52; 50', 52') größer ist als ein radial äußerer Durchmesser der Ausnehmung (54, 56; 54', 56').

11. Dichtung nach Anspruch 9 oder 10, **dadurch gekennzeichnet, dass** der Dichtungskörper (32; 32') im Bereich beider axialen Enden (34, 36; 34', 36') jeweils zumindest einen sich zumindest teilumfänglich erstreckenden Stützring (50, 52; 50', 52') aufweist.

12. Dichtung nach einem der Ansprüche 1 bis 11, **dadurch gekennzeichnet, dass** der Hohlraum (42; 42') an seinem radial äußeren Umfang einen sich zumindest teilumfänglich erstreckenden Ringspalt (60; 60') aufweist, und dass der zumindest eine Mittelstützring (58; 58'; 104) den Ringspalt (60; 60') im Wesentlichen verschließt.

13. Dichtung nach einem der Ansprüche 9 bis 12, **dadurch gekennzeichnet, dass** der oder die Stützringe (50, 52; 50', 52') und ggf. der Mittelstützring (58; 58'; 104) das Material PEEK und/oder ein Metall aufweisen.

14. Dichtung nach einem der Ansprüche 1 bis 13, **dadurch gekennzeichnet, dass** der Hohlraum (42; 42') über zumindest eine Öffnung (62; 62') kleinen Öffnungsquerschnitts mit dem Außenraum kommuniziert.

15. Dichtung nach Anspruch 14, **dadurch gekennzeichnet, dass** die zumindest eine Öffnung (62; 62') in dem zumindest einen Mittelstützring (56; 58') vorgesehen ist.

16. Dichtung nach einem der Ansprüche 1 bis 15, **dadurch gekennzeichnet, dass** das Dichtelement (38; 38'; 98) aus einem weichen elastomeren Material, vorzugsweise aus Silikon, gefertigt ist.

17. Dichtung nach einem der Ansprüche 1 bis 16, **dadurch gekennzeichnet, dass** der Dichtungskörper (32') im Durchgang für das Instrument ein Ventil (66'), insbesondere ein Lippenventil, aufweist.

18. Dichtung nach Anspruch 17, **dadurch gekennzeichnet, dass** das Ventil (66') einstückig mit dem radial inneren Wandbereich (44') des Dichtelements (38') ausgebildet ist.

19. Trokarhülse, mit einem Rohrschaft (14) und einem Trokarkopf (12; 72), der am proximalen Ende des Rohrschafts (14) angeordnet ist, sowie mit einer im Trokarkopf (12; 72) angeordneten Dichtung (30; 30'; 90) nach einem der Ansprüche 1 bis 18.

## Claims

1. A seal for a trocar sleeve (12), comprising a sealing body (32; 32'; 92) which, in the direction of a longitudinal centre axis (18; 18'; 78) of the sealing body (32; 32'; 92), has a first axial end (34; 34'; 94) and a second axial end (36; 36'; 96) and, between the first and second ends (34, 36; 34', 36'; 94, 96), an elastic sealing element (38; 38'; 98) which, in a radially inner area relative to the longitudinal centre axis (18; 18'; 78), defines a passage (40; 40'; 99) for an instrument, wherein the sealing element (38; 38'; 98) has a substantially tubular hollow space (42; 42'; 100) whose radially inner wall area (44; 44'; 102) annularly surrounds the passage (40; 40'; 99), and wherein the hollow space (42; 42'; 100) contains a fluid, wherein the sealing element (38; 38'; 98) has a radially outer wall area (48; 48') on the radially outer side of the hollow space (42; 42'; 100), a wall thickness of which is larger than the wall thickness of the radially inner wall area (44; 44'; 102), **characterized in that** in a central area, the sealing body (32; 32'; 92) is stiffened by at least one central support ring (58; 58'; 104) that at least partially surrounds the hollow space (42; 42'; 100) from the outside.

2. The seal of claim 1, **characterized in that** the fluid is a gas, in particular air.

3. The seal of claim 1, **characterized in that** the fluid is a liquid, preferably an oil.

4. The seal of claim 2, **characterized in that** the fluid is air and is at atmospheric pressure when the sealing element (38; 38'; 98) is in the relaxed state.

5. The seal of anyone of claims 1 through 4, **characterized in that** the radially inner wall area (44; 44'; 102) of the sealing element (38; 38'; 98) is bulged convexly, as seen from the longitudinal centre axis (18; 18'; 78), when said sealing element (38; 38'; 98) is in the relaxed state.

6. The seal of anyone of claims 1 through 5, **characterized in that** the hollow space (42; 42'; 100) is substantially O-shaped or oval in a section along the longitudinal centre axis (18; 18'; 78).

7. The seal of anyone of claims 1 through 6, **characterized in that** at least the radially inner wall area (44; 44'; 102) of the sealing element (38; 38'; 98) has a substantially uniform wall thickness.

8. The seal of anyone of claims 1 through 7, **characterized in that** the radially inner wall area (44; 44'; 102) of the sealing element (38; 38'; 98) has a thickened part (46; 46') in the shape of an annular bead that is directed radially further inwards.

9. The seal of anyone of claims 1 through 8, **characterized in that**, in the area of at least one of the axial ends (34, 36; 34', 36'), the sealing body (32; 32') is stiffened by at least one support ring (50, 52; 50', 52') that extends about at least a partial circumference.

10. The seal of claim 9, **characterized in that** the at least one support ring (50, 52; 50', 52') is received in a recess (54, 56; 54', 56') of the sealing body (32; 32'), an external diameter of the at least one support ring (50, 52; 50', 52') being greater than a radially outer diameter of the recess (54, 56; 54', 56').

11. The seal of claim 9 or 10, **characterized in that**, in the area of both axial ends (34, 36; 34', 36'), the sealing body (32; 32') in each case has at least one support ring (50, 52; 50', 52') that extends about at least a partial circumference.

12. The seal of any one of claims 1 through 11, **characterized in that** the hollow space (42; 42') has, on its radially outer circumference, an annular gap (60; 60') that extends about at least a partial circumference, and **in that** the at least one central support ring (58; 58'; 104) substantially closes the annular gap (60; 60').

13. The seal of anyone of claims 9 through 12, **characterized in that** the support ring or support rings (50, 52; 50', 52') and, if appropriate, the central support ring (58; 58'; 104) comprise the material PEEK and/or a metal.

14. The seal of anyone of claims 1 through 13, **characterized in that** the hollow space (42; 42') communicates with the exterior via at least one opening (62; 62') of small cross section.

15. The seal of claim 14, **characterized in that** the at least one opening (62; 62') is provided in the at least one central support ring (56; 58').

16. The seal of anyone of claims 1 through 15, **characterized in that** the sealing element (38; 38'; 98) is made of a soft elastomeric material, preferably of silicone.

17. The seal of anyone of claims 1 through 16, **characterized in that**, in the passage for the instrument, the sealing body (32') has a valve (66'), in particular a lip valve.

18. The seal of claim 17, **characterized in that** the valve (66') is formed in one piece with the radially inner wall area (44') of the sealing element (38').

19. A trocar sleeve, comprising a tubular shaft (14) and a trocar head (12; 72) which is arranged at the proximal end of the tubular shaft (14), and comprising a seal (30; 30'; 90) according to anyone of claims 1 through 18 arranged in the trocar head (12; 72).

## Revendications

1. Joint d'étanchéité pour une gaine de trocart (12), comprenant un corps d'étanchéité (32 ; 32' ; 92), qui présente, dans la direction d'un axe médian longitudinal (18, 18' ; 78) du corps d'étanchéité (32 ; 32' ; 92), une première extrémité axiale (34 ; 34' ; 94) et une deuxième extrémité axiale (36 ; 36' ; 96), et, entre la première et la deuxième extrémité (34, 36 ; 34' ; 36' ; 94, 96), un élément étanche (38 ; 38' ; 98) élastique, qui défnit, dans une zone radialement intérieure par rapport à l'axe médian longitudinal (18, 18' ; 78), un passage (40 ; 40' ; 99) pour un instrument, sachant que l'élément étanche (38 ; 38' ; 98) présente un espace creux (42 ; 42' ; 100) globalement de forme tubulaire, dont la zone de paroi radialement intérieure (44 ; 44' ; 102) entoure de manière à présenter une forme annulaire le passage (40 ; 40' ; 99), et sachant que l'espace creux (42 ; 42' ; 100) contient un fluide, sachant que l'élément étanche (38 ; 38' ; 98) présente une zone de paroi radialement extérieure (48 ; 48') de manière radiale en dehors de l'espace creux (42 ; 42' ; 100), dont l'épaisseur de paroi est plus grande que l'épaisseur de paroi de la zone de paroi radialement intérieure (44 ; 44' ; 102), **caractérisé en ce que** le corps d'étanchéité (32 ; 32' ; 92) est renforcé, dans une zone médiane, par au moins une bague d'appui centrale (58 ; 58' ; 104) entourant au moins en partie côté extérieur l'espace creux (42 ; 42' ; 100).

2. Joint d'étanchéité selon la revendication 1, **caractérisé en ce que** le fluide est un gaz, en particulier de l'air.

3. Joint d'étanchéité selon la revendication 1, **caractérisé en ce que** le fluide est un liquide, de préférence une huile.

4. Joint d'étanchéité selon la revendication 2, **caractérisé en ce que** le fluide est de l'air et se trouve à une pression ambiante lorsque l'élément étanche (38 ; 38' ; 98) est détendu.

5. Joint d'étanchéité selon l'une quelconque des revendications 1 à 4, **caractérisé en ce que** la zone de paroi radialement intérieure (44 ; 44' ; 102) de l'élément étanche (38 ; 38' ; 98) est bombée de manière convexe vue depuis l'axe médiane longitudinal (18 ; 18' ; 78) lorsque l'élément étanche est détendu.

6. Joint d'étanchéité selon l'une quelconque des revendications 1 à 5, **caractérisé en ce que** l'espace creux (42 ; 42' ; 100) présente essentiellement une forme en O ou est ovale dans une coupe le long de l'axe médiane longitudinal (18 ; 18' ; 78).

7. Joint d'étanchtéité selon l'une quelconque des revendications 1 à 6, **caractérisé en ce qu'**au moins la zone de paroi radialement intérieure (44 ; 44' ; 102) de l'élément étanche (38; 38' ; 98) présente une épaisseur de paroi globalement homogène.

8. Joint d'étanchéité selon l'une quelconque des revendications 1 à 7, **caractérisé en ce que** la zone de paroi radialement intérieure (44 ; 44' ; 102) de l'élément étanche (38 ; 38' ; 98) présente une surépaisseur (46 ; 46') en forme de bague à bourrelet orienté radialement davantage vers l'intérieur.

9. Joint d'étanchéité selon l'une quelconque des revendications 1 à 8, **caractérisé en ce que** le corps d'étanchéité (32 ; 32') est renforcé, dans la zone d'au moins une des extrémités axiales (34, 36 ; 34', 36'), par au moins une bague d'appui (50, 52 ; 50', 52') s'étendant au moins sur une partie de sa périphérie.

10. Joint d'étanchéité selon la revendication 9, **caractérisé en ce que** la au moins une bague d'appui (50, 52 ; 50', 52') est logée dans un évidement (54, 56 ; 54', 56') du corps d'étanchéité (32 ; 32'), sachant qu'un diamètre extérieur de la bague d'appui (50, 52 ; 50', 52') au moins au nombre de une est plus grand qu'un diamètre radialement extérieur de l'évidement (54, 56 ; 54', 56').

11. Joint d'étanchéité selon la revendication 9 ou 10, **caractérisé en ce que** le corps d'étanchéité (32 ; 32') présente, dans la zone des deux extrémités axiales (34, 36 ; 34', 36'), respectivement au moins une bague d'appui (50, 52 ; 50', 52') s'étendant au moins sur une partie de sa périphérie.

12. Joint d'étanchéité selon l'une quelconque des revendications 1 à 11, **caractérisé en ce que** l'espace creux (42 ; 42') présente, au niveau de sa périphérie radialement extérieure, une fente annulaire (60 ; 60') s'étendant au moins sur une partie de sa périphérie, et **en ce que** la au moins une bague d'appui centrale (58 ; 58' ; 104) obture globalement la fente annulaire (60 ; 60').

13. Joint d'étanchéité selon l'une quelconque des revendications 9 à 12, **caractérisé en ce que** la bague ou les bagues d'appui (50, 52 ; 50', 52') et éventuellement la bague d'appui centrale (58 ; 58' ; 104) présentent le matériau PEEK (polyétheréthercétone) et/ou un métal.

14. Joint d'étanchéité selon l'une quelconque des revendications 1 à 13, **caractérisé en ce que** l'espace creux (42 ; 42') communique avec l'espace extérieur par l'intermédiaire d'au moins une ouverture (62 ; 62') présentant une section transversale d'ouverture de petite dimension.

15. Joint d'étanchéité selon la revendication 14, **caractérisé en ce que** la au moins une ouverture (62 ; 62') est prévue dans la au moins une bague d'appui centrale (56 ; 58').

16. Joint d'étanchéité selon l'une quelconque des revendications 1 à 15, **caractérisé en ce que** l'élément étanche (38 ; 38' ; 98) est fabriqué à partir d'un matériau élastomère souple, de préférence à partir de silicone.

17. Joint d'étanchéité selon l'une quelconque des revendications 1 à 16, **caractérisé en ce que** le corps d'étanchéité (32') présente, dans le passage pour l'instrument, une soupape (66'), en particulier une soupape à bec.

18. Joint d'étanchéité selon la revendication 17, **caractérisé en ce que** la soupape (66') est réalisée d'un seul tenant avec la zone de paroi radialement intérieure (44') de l'élément étanche (38').

19. Gaine de trocart, comprenant une tige tubulaire (14) et une tête de trocart (12 ; 72), qui est disposée au niveau de l'extrémité proximale de la tige tubulaire (14), et comprenant également un joint d'étanchéité (30 ; 30' ; 90) selon l'une quelconque des revendications 1 à 18 disposé dans la tête de trocart (12 ; 72).
